# EUROPEAN PATENT APPLICATION

(11) **EP 2 614 781 A2**
(43) Date of publication of application: **17.07.2013**
(21) Application number: 12194753.5
(22) Date of filing: 29.11.2012
(51) Int. Cl.: A61B 17/04

(54) **Instrument tip having wire retention slot**

(30) Priority: 12.01.2012 US 201213349033
(71) Applicant: Microline Surgical, Inc, Beverly, MA 01915 (US)
(72) Inventor: Boulnois, Jean-Luc, Beverly, MA Massachusetts 01915 (US); Joshi, Sharad, Beverly, MA Massachusetts 01915 (US); Devlin, Chris A., Beverly, MA Massachusetts 01915 (US); Aliski, Peter, Beverly, MA Massachusetts 01915 (US); Ryll, Paul David, Beverly, MA Massachusetts 01915 (US)
(74) Representative: Schaumburg, Thoenes, Thurn, Landskron, Eckert

(57) **Abstract**

An instrument tip includes a wire retention chamber extending in a width direction of the tip and open at the sides of a tip, the wire retention chamber configured to removably retain a wire therein, and a wire retention slot extending from the outermost side of the tip to the wire retention chamber and in an oblique direction with respect to a longitudinal axis of the tip, the wire retention slot in open communication with the wire retention chamber, wherein the wire retention chamber has a diameter greater than a diameter of the wire retention slot.

## Description

### BACKGROUND

### 1. Field of the Invention

This invention relates to an instrument tip having a wire retention slot, and more particularly relates to an instrument tip having an enlarged wire retention chamber which prevents inadvertent dislodgement of the wire.

### 2. Background of the Invention

Medical procedures such as laparoscopy and the like, which employ a tip at the end of a tube for insertion into the patient, are beneficial because the incisions necessary to perform them are minimal in size, therefore promoting more rapid recovery and lower costs. For example, a patient who undergoes laparoscopic surgery may typically return to normal activity within a period of a few days to about a week, in contrast to more invasive procedures requiring a relatively larger incision (which may require about a month for recovery). Although the term "laparoscopic" is typically used hereinafter, such use of the term "laparoscopic" should be understood to encompass any such similar or related procedures such as, for example, natural orifice transluminal endoscopic surgery (NOTES), arthroscopic, endoscopic, pelvoscopic and/or thoroscopic or the like, in which relatively small incisions are used.

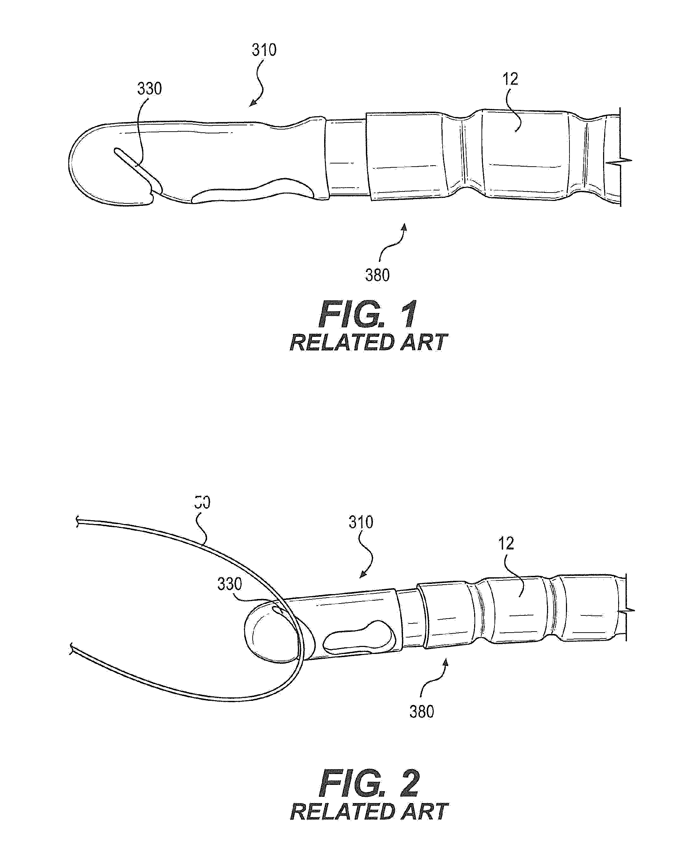

Gastric bypass procedures have become relatively commonplace. Referring to Figs. 1 and 2, which show a known bariatric tip 310, during such a gastric bypass procedure, a laparoscopic instrument 380 with bariatric tip 310 affixed to a shaft 12 is used to assist the surgeon in placing, securing, and tightening the gastric bypass band (not shown). Bands that have a suture 50 with a loop on the end require a tip that has a slot 330 capable of retrieving the loop so as to allow the surgeon to place and position the band. Known bariatric tip designs do not have a way of ensuring the loop will not inadvertently slip free during the tightening stage of the procedure.

The known bariatric tip 310 has a straight slot having a uniform diameter between slot walls and has no way of retaining the suture loop between the slot walls after engagement of the suture 50 by the tip. Rather, once the suture 50 is engaged, or captured, by the tip 310, the surgeon or other user must provide a proximal pulling force (thereby creating tension in the suture loop) to keep the suture at the bottom of the slot 330. Should the user cease pulling on the instrument 380 or should the user inadvertently push the instrument distally, there is a risk that the suture 50 could become disengaged from the tip 310, lengthening the procedure and risking complications to the patient while the surgeon attempts to re-engage the suture.

### SUMMARY OF THE INVENTION

Accordingly, a non-limiting feature of the disclosure provides a tip designed to retrieve a suture such as a suture loop found on gastric bypass band devices, enabling the surgeon to position and tighten the band. Thus, a non-limiting feature of the disclosure allows a suture or other thread or wire to be securely retained by the tip with or without pressure or other force being applied to the suture, thereby allowing the user a complete range of motion while positioning the band.
A non-limiting aspect of the disclosure provides an instrument tip having a wire retention chamber extending in a width direction of the tip and open at the sides of a tip, the wire retention chamber configured to removably retain a wire therein, and a wire retention slot extending from the outermost side of the tip to the wire retention chamber and in an oblique direction with respect to a longitudinal axis of the tip, the wire retention slot in open communication with the wire retention chamber, wherein the wire retention chamber has a diameter greater than a diameter of the wire retention slot.
In a non-limiting feature of the disclosure the wire retention slot has an exit restriction, the exit restriction having a diameter less than the diameter of the rest of the wire retention slot. Also, the diameter of the exit restriction may have a diameter generally equal to a diameter of the wire. The exit restriction may be located on the wire retention slot where the wire retention slot opens to the wire retention chamber.
In another non-limiting feature, the wire retention slot is offset from a center of the wire retention chamber where the wire retention slot opens to the wire retention chamber, in a height direction of the tip. Also, the wire retention slot may decrease in diameter in a direction toward the wire retention chamber. Further, the wire retention chamber may have a backstop where the wire retention slot opens to the wire retention chamber, the height of the backstop being at least half of the longest diameter of the wire retention chamber.
Another non-limiting aspect of the disclosure provides an instrument tip having a suture acceptance slot configured to accept a wire extending in a width direction of the tip and having opposed walls and extending from an outermost side of the tip in a direction oblique to a longitudinal axis of the tip, and a retention chamber at a terminal end of the suture acceptance slot, the retention chamber having a diameter larger than a distance between the opposed walls of the suture acceptance slot, in a direction orthogonal to the width direction of the tip, wherein the suture acceptance slot and retention chamber extend in the width direction of the tip and are open on opposite sides of the tip.
In a further non-limiting feature of the disclosure, a profile of the retention chamber may be generally oval in shape. Also, a proximal end of the tip may be configured to be removably affixed to a shaft of the instrument, and further may be configured to be threadably removably affixed to the shaft of the instrument. Further, the suture acceptance slot extends from the outermost side of the tip toward a distal end of the tip.
A further non-limiting aspect of the disclosure provides a wire placement instrument having a shaft and a tip, the tip being affixed to a distal end of the shaft, the tip comprising a tip slot, the tip slot configured to accept a wire extending in a width direction of the tip and having a wire retention slot extending radially inwardly from the outermost side of the tip, and a wire retention chamber at the bottom of the wire retention slot, the wire retention chamber configured to removably retain a wire therein, wherein the wire retention chamber has a diameter greater than a diameter of the wire retention slot.
In an additional non-limiting feature of the disclosure, the wire retention slot extends in an oblique direction with respect to a longitudinal axis of the tip. Further, the wire retention slot may distally extend in the oblique direction.
An additional non-limiting feature of the disclosure provides a method of operating a wire placement instrument, the method including accepting a wire in a wire retention slot of the instrument, moving the wire radially inwardly from the outermost side of the tip and into a a wire retention chamber at the bottom of the wire retention slot, and retaining a wire by the wire retention chamber, the wire retention chamber having a diameter greater than a diameter of the wire retention slot.

Other exemplary embodiments and advantages of the present invention may be ascertained by reviewing the present disclosure and the accompanying drawings, and the above description should not be considered to limit the scope of the present invention.

### BRIEF DESCRIPTION OF THE DRAWINGS

The present invention is further described in the detailed description which follows, in reference to the noted plurality of drawings, by way of non-limiting examples of preferred embodiments of the present invention, in which like characters represent like elements throughout the several views of the drawings, and wherein:

FIG. 1 shows an instrument tip in accordance with the related art;

FIG. 2 shows the instrument tip grasping a suture in accordance with the related art;

FIG. 3 shows an embodiment of an instrument tip in accordance with an aspect of the disclosure; and

FIG. 4 shows an embodiment of the instrument tip grasping a suture in accordance with an aspect of the disclosure.

### DETAILED DESCRIPTION

The particulars shown herein are by way of example and for purposes of illustrative discussion of the embodiments of the present invention only, and are presented for providing what is believed to be the most useful and readily understood description of the principles and conceptual aspects of the present invention. In this regard, no attempt is made to show structural details of the present invention in more detail than is necessary for the fundamental understanding of the present invention, the description taken with the drawings making apparent to those skilled in the art how the several forms of the present invention may be embodied in practice.

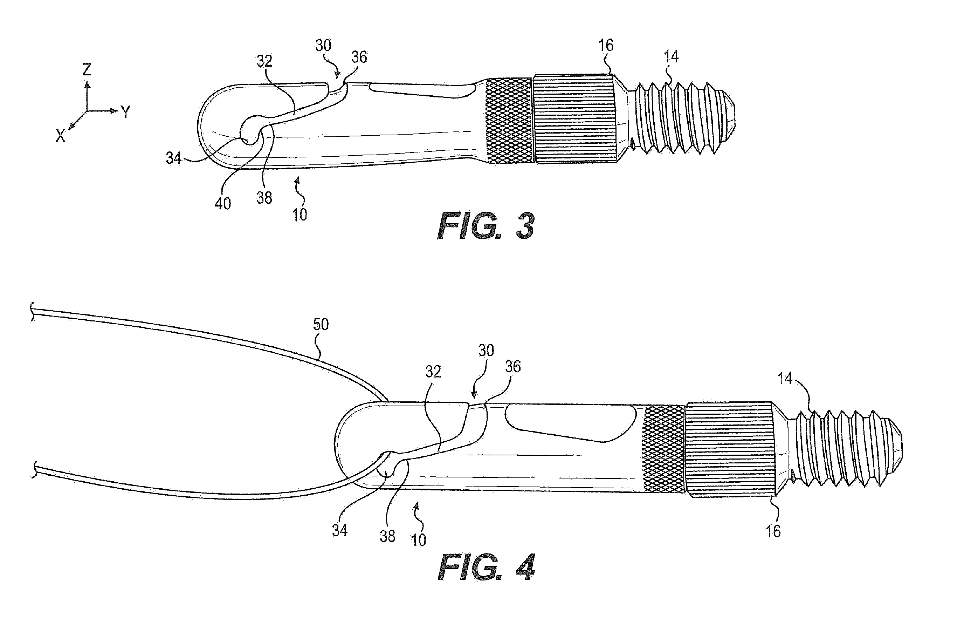

Referring to the drawings wherein like characters represent like elements, Figs. 3 and 4 shows an exemplary embodiment of in instrument tip 10 in accordance with a non-limiting aspect of the present disclosure. The tip 10 is preferably constructed of a metal, but those of skill in the art will appreciate that the tip can be constructed of any suitable material. The tip 10 is preferably removably attachable to shaft 12 of laparoscopic instrument 380, and may be removably attachable to the shaft 12 via threading 14 on a back hub 16 located at the proximal end of the tip (in the Z direction shown in Fig. 3), the back hub rotatable relative to the tip, although those skilled in the art will appreciate that the tip may be removably attachable to the shaft by other mechanisms, or that the tip may be integral or unitarily formed with the shaft. An exemplary shaft 12 to which the tip may be attached may be found in U.S. Patent Application No. 12/850,905, the contents of which are expressly incorporated by reference herein. In accordance with a non-limiting aspect, the distal end of the tip 10 is bull nosed to facilitate movement through a patient's body.

In accordance with a non-limiting aspect, the tip 10 is configured to retrieve and contain a suture 50 such as a suture loop, enabling the surgeon to position and tighten the band. While the present disclosure includes description with respect to a medical procedure, the present invention may be used in a variety of other, non-medical, environments, such as placing (instead of a suture) a wire, string, rope, belt, hose or any other type of narrow or flexible material in distant and/or hard-to- reach places, such as, e.g., placing a collar on a bear, or hanging up holiday lights. In a non-limiting embodiment, the tip is generally cylindrical in shape and has a generally circular cross section (taken along a plane in the X-Z direction shown in Fig. 3), and is affixed to a shaft having a similar cross section, although those skilled in the art would appreciate that the tip can take other suitable shapes.

The tip 10 includes a slot 30 for receiving the suture (which may be machined into the body of the tip), and the slot extends the entire width of the tip (the X direction shown in Fig. 3) such that the slot in open on opposite sides of the tip. The slot 30 is primarily divided up into two sections, namely, a wire retention slot 32 and a wire retention chamber 34. At the outermost side of the tip, the wire retention slot 32 includes an entrance guide slot 36 where the suture 50 enters the wire retention slot. The wire retention slot 32 terminates at and is in open communication with the wire retention chamber 34. In accordance with a non-limiting feature of the disclosure, the wire retention slot 32 is curved and the distance between opposed walls of the wire retention slot (i.e., the diameter) becomes smaller toward the wire retention chamber 34. The wire retention slot 32 is shown distally extending toward the wire retention chamber 34 and in a direction oblique to the a longitudinal axis direction of the tip (*i.e.,* a direction oblique to the Y direction shown in Fig. 3), since this is the preferred configuration when performing a gastric bypass procedure; however, it is appreciated by those skilled in the art that the wire retention slot may proximally extend toward the wire retention chamber (*i.e.,* such that the wire retention chamber is located proximally (in the Y direction shown in Fig. 3) of the entrance guide slot 36), or that the wire retention slot may radially extend toward a radial center of the tip 10 (*i.e.,* in the Z direction shown in Fig. 3).

In accordance with a non-limiting feature of the disclosure, the narrowest space between the opposed walls of the wire retention slot 32 is referred to as exit restriction 38 and is located on the wire retention slot where the wire retention slot opens to the wire retention chamber 34 (*i.e.,* the portion of the wire retention slot located furthest from the outside of the tip in the Z direction of Fig. 3), although those skilled in the art will appreciate that the exit restriction 38 may be elsewhere along the wire retention slot 32. In accordance with a non-limiting feature, the exit restriction 38 is approximately or exactly the same size as the wire or suture 50 to be applied, in order to prevent inadvertent migration of the suture from the wire retention chamber 34. The type of suture to be used is preferably a 2-0 suture, although those of skill in the art will appreciate that bands, strings, wires and the like of other sizes may be used in alternative embodiments. Additionally, in a non-limiting embodiment, the diameter of the tip 10 and shaft 12 (at their widest points and taken along a plane in the X-Z direction shown in Fig. 3) is 5 millimeters, those skilled in the art will appreciate that the tip 10 can be larger or smaller than 5 millimeters in alternative embodiments. While it is not necessary for the exit restriction 38 to continuously extend the entire width of the tip (in the X direction shown in Fig. 3)(for example, the exit restriction may include one or more bosses), it is suggested that the exit restriction be free of sharp edges to avoid severing the suture 50.

It is not required that the wire retention chamber 34 have any particular shape, only that it have a diameter (taken along a plane in the X-Z direction shown in Fig. 3) larger than the distance between the opposed walls of the wire retention slot 32, such that the suture 50 can "float" within the wire retention chamber, although in accordance with a non-limiting feature, the wire retention chamber 34 does not taper toward the wire retention slot 32, in order to avoid accidental migration of the suture 50 from the wire retention chamber to the wire retention slot. Thus, the profile of the wire retention chamber 34 (in a plane in the Y-Z direction shown in Fig. 3) may be oval, circular, polygonal, hourglass-shaped, kidney-shaped and the like, or any other suitable shape. Also in a non-limiting embodiment, the profile of the wire retention chamber (and the wire retention slot 32 as well) uniformly and prismatically extends the width of the tip (in the X direction shown in Fig. 3). In other words, the dimensions of the slot 30 are uniform along width direction of the tip (in the X direction shown in Fig. 3), although the wire retention chamber may form an arc (in the X-Y plane as shown in Fig. 3) to accommodate and correspond to the arc of a bent wire or suture loop 50 as shown in Fig. 3.

In accordance with a non-limiting feature, the location where the wire retention chamber 34 where the retention chamber 34 exits to the wire retention slot 32 (*i.e.,* the intersection of the wire retention slot and the wire retention chamber) is offset from the center of the wire retention chamber, creating a wall-like backstop 40. In a non-limiting embodiment, the backstop 40 is preferably greater in height in the Z direction than the diameter of the wire retention chamber 34 in the Z direction. The backstop 40 may be contoured or flat, and functions to engage the suture 50 and to prevent the suture from entering the wire retention slot 32. In accordance with a non-limiting feature, during movement of the suture 50 within the wire retention chamber 34, due to the convoluted space of the wire retention chamber including the backstop 40, the suture is more likely to contact one of the walls of the wire retention chamber than enter the wire retention slot. In the event that the suture 50 begins to enter the wire retention slot, the exit restriction 38 further prevents unwanted migration of the suture out of the slot 30. Thus, the suture 50 is securely held within the tip 10 in the absence of proximal pulling force.

A description of a method of capturing and releasing the suture 50 from the tip is provided below. To capture the suture 50, the surgeon distally moves the tip 10 (which is affixed to an instument12) underneath and behind the suture (or moves the tip inside the looped suture, in the event that the suture is in a loop) and moves the tip such that the suture moves into the entrance guide slot 36. Once the suture is in the entrance guide slot 36, the surgeon proximally pulls the tip so that the suture 50 moves within the wire retention slot (*i.e.,* the wire retention slot is pulled across the suture) until the suture slides all the way down into the wire retention chamber 34, where the suture is retained within the tip. To disengage the tip 10 from the suture 50, the surgeon distally moves the tip with sufficient force that the suture exits the wire retention chamber 34 and enters the wire retention slot 32 and passes over the exit restriction 38. Once the suture 50 passes over the exit restriction 38, the surgeon continues to distally push the tip so that the suture slides up the wire retention slot 32 until the suture exits the tip through the entrance guide slot 36.

In view of the foregoing, the present disclosure, through one or more of its various aspects, embodiments and/or specific features or sub-components, is thus intended to bring out one or more of the advantages as specifically noted below.

The illustrations of the embodiments described herein are intended to provide a general understanding of the structure of the various embodiments. The illustrations are not intended to serve as a complete description of all of the elements and features of apparatus and systems that utilize the structures or methods described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

Unless otherwise defined, all technical and scientific terms used herein have the same meaning as commonly understood by one of ordinary skill in the art to which this invention belongs. The terminology used in the description of the invention herein is for describing particular embodiments only and is not intended to be limiting of the invention. As used in the description of the invention and the appended claims, the singular forms "a," "an," and "the" are intended to include the plural forms as well, unless the context clearly indicates otherwise. All publications, patent applications, patents, and other references mentioned herein are expressly incorporated by reference in their entirety.

Unless otherwise indicated, all numbers expressing quantities of ingredients and the like used in the specification and claims are to be understood as being modified in all instances by the term "about." The term "generally" as used herein is to be understood as an approximation allowing for variations while still maintaining the efficacy of the invention. Accordingly, unless indicated to the contrary, the numerical and geometric parameters and relationships set forth in the following specification and attached claims are approximations that may vary depending upon the desired properties sought to be obtained by the present invention. At the very least, and not as an attempt to limit the application of the doctrine of equivalents to the scope of the claims, each numerical and geometric parameters and relationship should be construed in light of the number of significant digits and ordinary rounding approaches.

The Abstract of the Disclosure is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents, and shall not be restricted or limited by the foregoing detailed description.

## Claims

1. An instrument tip, comprising:
a wire retention chamber extending in a width direction of the tip and open at the sides of a tip, the wire retention chamber configured to removably retain a wire therein; and
a wire retention slot extending from the outermost side of the tip to the wire retention chamber and in an oblique direction with respect to a longitudinal axis of the tip, the wire retention slot in open communication with the wire retention chamber, wherein the wire retention chamber has a diameter greater than a diameter of the wire retention slot.

2. The instrument tip according to claim 1, wherein the wire retention slot comprises an exit restriction, the exit restriction having a diameter less than the diameter of the rest of the wire retention slot.

3. The instrument tip according to claim 2, wherein the diameter of the exit restriction has a diameter generally equal to a diameter of the wire.

4. The instrument tip according to claim 2, wherein the exit restriction is located on the wire retention slot where the wire retention slot opens to the wire retention chamber.

5. The instrument tip according to claim 1, wherein the wire retention slot is offset from a center of the wire retention chamber where the wire retention slot opens to the wire retention chamber, in a height direction of the tip.

6. The instrument tip according to claim 1, wherein the wire retention slot decreases in diameter in a direction toward the wire retention chamber.

7. The instrument tip according to claim 1, wherein the wire retention chamber comprises a backstop where the wire retention slot opens to the wire retention chamber, the height of the backstop being at least half of the longest diameter of the wire retention chamber.

8. An instrument tip comprising:
a suture acceptance slot configured to accept a wire extending in a width direction of the tip and having opposed walls and extending from an outermost side of the tip in a direction oblique to a longitudinal axis of the tip; and
a retention chamber at a terminal end of the suture acceptance slot, the retention chamber having a diameter larger than a distance between the opposed walls of the suture acceptance slot, in a direction orthogonal to the width direction of the tip, wherein the suture acceptance slot and retention chamber extend in the width direction of the tip and are open on opposite sides of the tip.

9. The instrument tip according to claim 8, wherein a profile of the retention chamber is generally oval in shape.

10. The instrument tip according to claim 8, wherein a proximal end of the tip is configured to be removably affixed to a shaft of the instrument.

11. The instrument tip according to claim 10, wherein the proximal end of the tip is configured to be threadably removably affixed to the shaft of the instrument.

12. The instrument tip according to claim 8, wherein the suture acceptance slot extends from the outermost side of the tip toward a distal end of the tip.

13. A wire placement instrument comprising:
a shaft; and
a tip affixed to a distal end of the shaft, the tip comprising a tip slot, the tip slot configured to accept a wire extending in a width direction of the tip and comprising:
a wire retention slot extending radially inwardly from the outermost side of the tip; and
a wire retention chamber at the bottom of the wire retention slot, the wire retention chamber configured to removably retain a wire therein, wherein the wire retention chamber has a diameter greater than a diameter of the wire retention slot.

14. The wire placement instrument according to claim 13, wherein the wire retention slot extends in an oblique direction, with respect to a longitudinal axis of the tip.

15. The wire placement instrument according to claim 14, wherein the wire retention slot distally extends in the oblique direction.

16. The wire placement instrument according to claim 13, wherein the tip is removably affixed to the distal end of the shaft.

17. The wire placement instrument according to claim 16, wherein the tip is threadably removably affixed to the distal end of the shaft.

18. The wire placement instrument according to claim 13, wherein the wire retention slot comprises an exit restriction, the exit restriction having a diameter less than the diameter of the rest of the wire retention slot.

19. The wire placement instrument according to claim 13, wherein:
the wire retention chamber comprises a backstop where the wire retention slot meets the wire retention chamber, and
the height of the backstop is at least half of the longest diameter of the wire retention chamber.

20. A method of operating a wire placement instrument, the method comprising:
accepting a wire in a wire retention slot of the instrument;
moving the wire radially inwardly from the outermost side of the tip and into a a wire retention chamber at the bottom of the wire retention slot; and
retaining a wire by the wire retention chamber, the wire retention chamber having a diameter greater than a diameter of the wire retention slot.
